(19) Europäisches Patentamt
European Patent Office
Office européen des brevets

(11) EP 0 959 065 B1

(12) EUROPEAN PATENT SPECIFICATION

(45) Date of publication and mention of the grant of the patent:
**03.04.2002 Bulletin 2002/14**

(51) Int Cl.[7]: **C07C 69/743**, A01N 53/00

(21) Application number: **99108913.7**

(22) Date of filing: **05.05.1999**

(54) **Pyrethroid compound and composition for controlling pest containing the same**
Pyrethroidverbindung und diese enthaltende pestizide Zusammensetzung
Composé pyréthroide et composition pesticide le contenant

(84) Designated Contracting States:
**IT**

(30) Priority: **22.05.1998 JP 14155098**

(43) Date of publication of application:
**24.11.1999 Bulletin 1999/47**

(73) Proprietor: **SUMITOMO CHEMICAL COMPANY, LIMITED**
**Chuo-ku, Osaka (JP)**

(72) Inventors:
- **Mori, Tatsuya**
**Toyonaka-shi (JP)**
- **Ishiwatari, Takao**
**Toyonaka-shi (JP)**

(74) Representative: **Henkel, Feiler, Hänzel**
**Möhlstrasse 37**
**81675 München (DE)**

(56) References cited:
**EP-A- 0 031 199**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 959 065 B1

## Description

The present invention relates to a pyrethroid compound and a composition for controlling pest containing said compound as an active ingredient.

BACKGROUND OF THE INVENTION

It has been known that pyrethroid compounds obtained by using benzyl alcohol substituted with fluorine atoms as an alcohol component have insecticidal activity, and that, of their geometrical isomers, the cis-isomers are highly active (EP-A-31199). One of the cis-isomers, 2,3,5,6-tetrafluoro-4-methylbenzyl (Z)-(1RS)-cis-3-(2-chloro-3,3,3-trifluoroprop-1-enyl)-2,2-dimethyl cyclopropanecarboxylate, has already been put to practical use for controlling soil insect pests.

Application of such compositions containing a pyrethroid compound for controlling pests, however, is often unavoidably limited not only by their controlling effect but also by their safety, depending on the application conditions. In particular, usually, compositions for controlling insect pest for preventing epidemics are used mainly in a limited space in a living environment and hence are required to have a desirable insecticidal activity as well as a low toxicity to mammals such as human beings, livestock and pets.

BRIEF SUMMARY OF THE INVENTION

In recognition of the situation, the present inventors have earnestly investigated and consequently found that a specific optically active, trans-isomer of 4-methyl-2,3,5,6-tetrafluorobenzyl 3-(2,2-dichlorovinyl)-2,2-dimethyl-cyclopropanecarboxylate has a high activity to control pest and a high safety factor to mammals, to accomplish the present invention.

Thus, the present invention relates to 4-methyl-2,3,5,6-tetrafluorobenzyl (1R)-trans-3-(2,2-dichlorovinyl)-2,2-dimethylcyclopropanecarboxylate (hereinafter referred to as the present compound) represented by the formula,

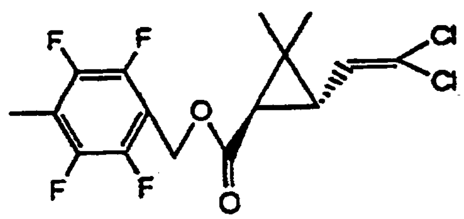

and a composition for controlling pest containing the present compound as an active ingredient.

DETAILED DESCRIPTION OF THE INVENTION

The present compound can be produced, for example, by reacting 4-methyl-2,3,5,6-tetrafluorobenzyl alcohol (hereinafter referred to as said alcohol compound) represented by the formula,

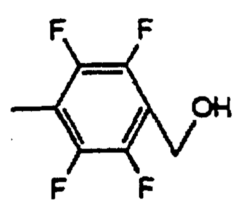

with (1R)-trans-3-(2,2-dichlorovinyl)-2,2-dimethylcyclopropanecarboxylic acid (hereinafter referred to as said carboxylic acid) represented by the formula,

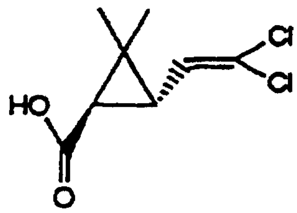

or a reactive derivative of said carboxylic acid (e.g. the acid halide and acid anhydride).

The reaction is usually carried out in a solvent in the presence of a condensing agent or a base.

The condensing agent includes, for example, dicyclohexylcarbodiimide (DCC) and 1-ethyl-3-(3-dimethylaminopropyl)carbodiimide hydrochloride (WSC).

The base includes, for example, organic bases such as triethylamine, pyridine, N,N-diethylaniline, 4-dimethylaminopyridine and diisopropylethylamine.

The solvent includes, for example, hydrocarbons such as benzene, toluene and hexane; ethers such as diethyl ether and tetrahydrofuran; and halogenated hydrocarbons such as dichloromethane and 1,2-dichloroethane.

The reaction time ranges usually from 5 minutes to 72 hours.

The reaction temperature ranges usually from -20°C to +100°C (or from -20°C to the boiling point of the solvent in the case where the boiling point of the solvent is lower than +100°C), preferably from -5°C to +100°C (or from -5°C to the boiling point of the solvent in the case where the boiling point of the solvent is lower than +100°C).

Although the molar ratio of said alcohol compound to said carboxylic acid or a reactive derivative of said carboxylic acid used can be selected without any limitation, it is preferable to carry out the reaction by using them in an approximate molar ratio of 1:1.

The condensing agent or the base can be used in any amount of usually 1 mole to excess mole, preferably 1 mole to 5 moles, per mole of said alcohol compound.

The present compound can be obtained by subjecting the reaction solution after completion of the reaction to conventional after-treatments such as extraction with an organic solvent and concentration. If necessary, it can be further purified by conventional operations such as chromatography, distillation and recrystallization.

Said alcohol compound and said carboxylic acid can be produced according to, for example, the process disclosed in EP-A-31199.

Examples of pests on which the present compound has controlling effect are the arthropods given below. Because the present compound can be used as an active ingredient of a composition for controlling the following arthropods and in particular is superior in safety factor, it is especially effective as the active ingredient of a composition for controlling pest for preventing epidemics, which is required to have a controlling effect as well as a high safety to mammals.

Lepidoptera

Pyralidae (pyralid moths) such as Chilo suppressalis (rice stem borer), Cnaphalocrocis medinalis (rice leafroller) and Plodia interpunctella (Indian meal moth) ; Noctuidae such as Spodoptera litura (tobacco cutworm), Pseudaletia separata (rice armyworm) and Mamestra brassicae (cabbage armyworm); Pieridae such as Pieris rapae crucivora (common cabbageworm); Tortricidae (tortricid moths) such as Adoxophyes spp.; Carposinidae; Lyonetiidae (lyonetiid moths); Lymantriidae (tussock moths); Antographa spp.; Agrotis spp. such as Agrotis segetum (turnip cutworm) and Agrotis ipsilon (black cutworm); Helicoverpa spp.; Heliothis spp.; Plutella xylostella (diamondback moth); Parnara guttata (rice skipper); Tinea pellionella (casemaking clothes moth); Tineola bisselliella (webbing clothes moth); etc.

Diptera

Culex spp. such as Culex pipiens pallens (common mosquito)and Culex tritaeniorhynchus; Aedes spp. such as Aedes aegypti and Aedes albopictus; Anopheles spp. such as Anopheles sinensis; Chironomidae midges; Muscidae such as Musca domestica (housefly), Muscina stabulans (false stablefly) and Fannia canicularis (little housefly); Calliphoridae; Sarcophagidae; Anthomyiidae (anthomylid flies) such as Delia platura (seedcorn maggot) and Delia antiqua (onion maggot); Tephritidae (fruit flies); Drosophilidae (small fruit flies, vinegar flies); Psychodidae (moth flies, sand flies); Simuliidae (black flies); Tabanidae; Stomoxyidae (stable flies); biting midges; etc.

Dictyoptera

Blattella germanica (German cockroach), Periplaneta fuliginosa (smokybrown cockroach), Periplaneta americana (American cockroach), Periplaneta brunnea (brown cockroach), Blatta orientalis (oriental cockroach), etc.

Hymenoptera

Formicidae (ants); Vespidae (hornets); Bethylidae (bethylid wasps); Tenthredinidae (sawflies) such as Athalia rosae ruficornis (cabbage sawfly); etc.

Siphonaptera

Ctenocephalides canis, Ctenocephalides felis, Pulex irritans, etc.

Anoplura

Pediculus humanus, Phthirus pubis, etc.

Isoptera termites

Reticulitermes speratus, Coptotermes formosanus (Formosan subterranean termite), etc.

Acarina (mites and ticks)

Pyroglyphidae such as Dermatophagoides farinae and Dermatophagoides pteronyssinus; Acaridae such as Tyrophagus putrescentiae Schrank (mold mite, copra mite, forage mite) and Aleuroglyphus ovatus Troupeau (brown legged grain mite); Glycyphagidae such as Glycyphagus privatus, Glycyphagus domesticus and Glycyphagus destructor Schrank (groceries mite); Cheyletidae such as Cheyletus malaccensis and Cheyletus moorei; Tarsonemidae; Chrtoglyphus; Oribatei; Tetranychidae (spider mites) such as Tetranychus urticae (two-spotted spider mite), Tetranychus kanzawai (Kanzawa spider mite), Panonychus citri (citrus red mite) and Panonychus ulmi (European red mite); Ixodidae such as Haemaphysalis longicornis; etc.

Hemiptera

Delphacidae (planthoppers) such as Laodelphax striatellus (small brown planthopper), Nilaparvata lugens (brown planthopper) and Sogatella furcifera (white-backed rice planthopper); Deltocephalidae (leaf-hoppers) such as Nephotettix cincticeps (green rice leafhopper) and Nephotettix virescens (green rice leafhopper); Aphididae (aphids); Pentatomidae (bugs); Aleyrodidae (whiteflies); Coccidae (scales); Tingidae (lace bugs); Psyllidae (psyllids); etc.

Coleoptera

Attagenus unicolor; Anthrenus verbasci (varied carpet beetle); corn rootworms such as Diabrotica virgifera (western corn rootworm) and Diabrotica undecimpunctaca howardi (southern corn rootworm); Scarabaeidae (scarabs) such as Anomala cuprea (cupreous chafer) and Anomala rufocuprea (soybean beatle); Curculionidae (weevils) such as Sitophilus zeamais (maize weevil), Lissorhoptrus oryzophilus (ricewater weevil), Anthonomus grandis grandis (boll weevil) and Callosobruchus chinensis (adzuki bean weevil); Tenebrionidae (darkling beetles) such as Tenebrio molitor (yellow mealworm) and Tribolium castaneum (red fluor beetle); Chrysomelidae (corn rootworms) such as Oulema oryzae (rice leaf beetle), Phyllotreta striolata (striped flea beetles) and Aulacophora femoralis (cucurbit leaf beetle); Anobiidae; Epilachna spp. such as Henosepilachna vigintioctopunctata (twenty-eight-spotted ladybirds); Lyctidae (powder post beetles); Bostrychidae (false powder post beetles); Cerambycidae; Paederus fuscipes (robe beetle); etc.

Thysanoptera (thrips)

Thrips palmi, Thrips hawaiiensis (flower thrips), etc.

Orthoptera

Gryllotalpidae (mole crickets), Acrididae (grasshoppers), etc.

In the present invention, the composition for controlling pest is for controlling arthropods such as insects and/or acarines.

When the present compound is used as an active ingredient of the composition for controlling pest, the present compound is applied usually after having been formulated into various formulations, for example, oil formulations; emulsifiable concentrates; wettable powders; flowable concentrates such as aqueous suspension concentrates or aqueous emulsion concentrates; granules; dusts; aerosols; heating fumigants such as mosquito coils, electric mosquito mats, or solutions for heating fumigation using an absorbent wick; heating smoking formulations such as self-burning-type smoking formulations, chemical-reaction-type smoking formulations, or electrically heating-type smoking formulations using a porous ceramic plate; non-heating volatile formulations such as resin volatile formulations, or impregnated paper volatile formulations; foggings; ULV formulations; poisonous baits; or the like, either by mixing the present compound or a solution thereof with a solid carrier, liquid carrier, gaseous carrier, base material for poisonous bait, or base material for mosquito coil, and optionally adding auxiliaries for formulation such as surfactants, or by impregnating a base material such as a mosquito coil or mat with the present compound or a solution thereof.

These formulations usually contain the present compound as an active ingredient in an amount of 0.001 to 95% by weight.

The solid carrier used for formulation includes, for example, fine powders and granules of clays (e.g. kaolin clay, diatomaceous earth, synthetic hydrated silicon dioxide, bentonite, fubasami clay and acid clay), talcs, ceramics, and other inorganic minerals (e.g. sericite, quartz, activated carbon, calcium carbonate and hydrated silica). The liquid carrier includes, for example, water, alcohols (e.g. methanol and ethanol), ketones (e.g. acetone and methyl ethyl ketone), aromatic hydrocarbons (e.g. benzene, toluene, xylene, ethylbenzene and methylnaphthalene), aliphatic hydrocarbons (e.g. hexane, cyclohexane, kerosene and light oil), esters (e.g. ethyl acetate and butyl acetate), nitriles (e.g. acetonitrile and isobutyronitrile), ethers (e.g. diisopropyl ether and dioxane), acid amides (e.g. N,N-dimethylformamide and N,N-dimethylacetamide), halogenated hydrocarbons (e.g. dichloromethane, trichloroethane and carbon tetrachloride), dimethyl sulfoxide, and vegetable oils (e.g. soybean oil and cotton seed oil). The gaseous carrier, so-called propellant, includes, for example, CFC gas, butane gas, LPG (liquefied petroleum gas), dimethyl ether and carbon dioxide.

The surfactant includes, for example, alkyl sulfates, alkylsulfonates, alkylarylsulfonates, alkyl aryl ethers and their polyoxyethylenated products, polyethylene glycol ethers, polyhydric alcohol esters and sugar alcohol derivatives.

The auxiliaries for formulation such as adhesive agents and dispersants include, for example, casein, gelatin, polysaccharides (e.g. starch powder, gum arabic, cellulose derivatives and alginic acid), lignin derivatives, bentonite, saccharides, and synthetic water-soluble polymers [e.g. poly(vinyl alcohol)s, poly(vinyl-pyrrolidone)s and poly(acrylic acid)s]. The stabilizer includes, for example, PAP (acidic isopropyl phosphate), BHT (2,6-di-tert-butyl-4-methylphenol), BHA (a mixture of 2-tert-butyl-4-methoxyphenol and 3-tert-butyl-4-methoxyphenol), vegetable oils, mineral oils, surfactants, and fatty acids or their esters.

Base materials for mosquito coils include, for example, mixtures of vegetable powders (e.g. wood powder and Pyrethrum marc) and binders (e.g. Tabu powder, starch and gluten).

Base materials for electric mosquito mats include, for example, plates obtained by coagulating fibrils of cotton linter or a mixture of cotton linter and pulp.

Base materials for self-burning-type smoking formulations include, for example, combustible and heat-generating agents (e.g. nitrates, nitrites, guanidine salts, potassium chlorate, nitrocellulose, ethylcellulose and wood powder), pyrolysis-promoting agents (e.g. alkali metal salts, alkaline earth metal salts, dichromates and chromates), oxygen-supplying agents (e.g. potassium nitrate), combustion-supporting agents (melamine and wheat starch), extending agents (e.g. diatomaceous earth) and binders (e.g. synthetic pastes).

Base materials for the chemical-reaction-type smoking formulations include, for example, heat-generating agents (e.g. sulfides, polysulfides, hydrosulfides and hydrate salts of alkali metals, and calcium oxide), catalysts (e.g. carbonaceous substances, iron carbide and activated clay), organic foaming agents (e.g. azodicarbonamide, benzenesulfonylhydrazide, dinitrosopentamethylenetetramine, polystyrenes and polyurethanes) and fillers (e.g. natural fiber pieces and synthetic fiber pieces).

Base materials for the non-heating volatile formulations include, for example, thermoplastic resins, filter papers and Japanese papers.

The base material for poisonous bait includes, for example, bait components (e.g. cereal flour, vegetable oils, saccharides and crystalline cellulose), antioxidants (e.g. dibutylhydroxytoluene and nordihydroguaiaretic acid), preservatives (e.g. dehydroacetic acid), agents for preventing consumption by children or pets (e.g. red pepper powder) and attractants (e.g. cheese perfume, onion perfume and peanut oil).

The flowable concentrates (aqueous suspension concentrates or aqueous emulsion concentrates) usually comprise the present compound, a dispersant, a suspension assistant (e.g. a protective colloid or a compound capable of imparting thixotropic properties), suitable auxiliaries (e.g. defoaming agents, rust preventives, stabilizers, spreaders, penetration assistants, anti-freezing agents, bactericides, and fungicides) and water. The protective colloid includes,

for example, gelatin, casein, gums, cellulose ether and poly(vinyl alcohol)s. The compound capable of imparting thixotropic properties includes, for example, bentonite, aluminum magnesium silicate, xanthan gum and poly(acrylic acid) s. It is also possible to prepare an oil-based suspension concentrate by using an oil substantially incapable of dissolving the present compound, in place of water.

The formulations thus obtained are applied as they are or after diluted with water or the like, depending on the purposes.

It is also possible to apply the formulations in admixture or combination with other insecticides, acaricides, repellents, synergists or the like.

The insecticides and acaricides include, for example, organophosphorus compounds such as Fenitrothion [O,O-dimethyl O-(3-methyl-4-nitrophenyl)phosphorothioate], Fenthion [O,O-dimethyl O-(3-methyl-4-(methylthio)phenyl)-phosphorothioate], Diazinon [O,O-diethyl O-2-isopropyl-6-methylpyrimidin-4-ylphosphorothioate], Chlorpyrifos [O,O-diethyl 0-3,5,6-trichloro-2-pyridylphosphorothioate], Acephate [O,S-dimethylacetylphosphoramidothioate], Methidathion [S-2,3-dihydro-5-methoxy-2-oxo-1,3,4-thiadiazol-3-ylmethyl O,O-dimethylphosphorodithioate], Disulfoton [O,O-diethyl S-2-ethylthioethylphosphorodithioate], DDVP [2,2-dichlorovinyldimethyl phosphate], Sulprofos [O-ethyl O-4-(methylthio)phenyl S-propylphosphorodithioate], Cyanophos [O-4-cyanophenyl O,O-dimethylphosphorothioate], Dioxabenzophos [2-methoxy-4H-1,3,2-benzodioxaphosphorine-2-sulfide], Dimethoate [O,O-dimethyl S-(N-methylcarbamoylmethyl) dithiophosphate], Phenthoate [ethyl 2-dimethoxyphosphinothioylthio(phenyl)acetate], Malathion [diethyl (dimethoxyphosphinothioylthio)succinate], Trichlorfon [dimethyl 2,2,2,-trichloro-1-hydroxyethylphosphonate], Azinphosmethyl [S-3,4-dihydro-4-oxo-1,2,3-benzotriazin-3-ylmethyl O,O-dimethylphosphorodithioate], Monocrotophos [dimethyl (E)-1-methyl-2-(methylcarbamoy)vinylphosphate], Ethion [O,O,O',O'-tetraethyl S,S'-methylenebis-(phosphorodithioate)]; carbamate type compounds such as BPMC [2-sec-butylphenylmethylcarbamate], Benfuracarb [ethyl N-[2,3-dihydro-2,2-dimethylbenzofuran-7-yloxycarbonyl(methyl)aminothio]-N-isopropyl-β-alaninate], Propoxur [2-isopropoxyphenyl N-methylcarbamate], Carbosulfan [2,3-dihydro-2,2-dimethyl-7-benzo[b]furanyl N-dibutylaminothio-N-methylcarbamate], Carbaryl [1-naphthyl-N-methylcarbamate], Methomyl [S-methyl-N-[(methyl-carbamoyl)oxy] thioacetimidate], Ethiofencarb [2-(ethylthiomethyl)phenylmethylcarbamate], Aldicarb [2-methyl-2-(methylthio)propionaldehyde O-methylcarbamoyloxime], Oxamyl [N,N-dimethyl-2-methylcarbamoyloxyimino-2-(methylthio) acetamide], Phenothiocarb [(S-4-phenoxybutyl)-N,N-dimethylthiocarbamate]; pyrethroid compounds such as Etofenprox [2-(4-ethoxyphenyl)-2-methylpropyl-3-phenoxybenzyl ether], Fenvalerate [(RS)-α-cyano-3-phenoxybenzyl (RS)-2-(4-chlorophenyl)-3-methylbutyrate], Esfenvalerate [(S)-α-cyano-3-phenoxybenzyl (S)-2-(4-chlorophenyl)-3-methylbutyrate], Fenpropathrin [(RS)-α-cyano-3-phenoxybenzyl 2,2,3,3-tetramethylcyclopropanecarboxylate], Cypermethrin [(RS)-α-cyano-3-phenoxybenzyl (1RS)-cis, trans-3-(2,2-dichlorovinyl)-2,2-dimethylcyclopropanecarboxylate], Permethrin [3-phenoxybenzyl (1RS)-cis,trans-3-(2,2-dichlorovinyl)-2,2-dimethylcyclopropanecarboxylate], Cyhalothrin [(RS)-α-cyano-3-phenoxybenzyl (Z)-(1RS)-cis-3-(2-chloro-3,3,3-trifluoroprop-1-enyl)-2,2-dimethylcyclopropanecarboxylate], Deltamethrin [(S)-α-cyano-3-phenoxybenzyl (1R)-cis-3-(2,2-dibromovinyl)-2,2-dimethylcyclopropanecarboxylate], Cycloprothrin [(RS)-α-cyano-3-phenoxybenzyl (RS)-2,2-dichloro-1-(4-ethoxyphenyl)cyclopropanecarboxylate], Fluvalinate [α-cyano-3-phenoxybenzyl N-(2-chloro-α,α,α-trifluoro-p-tolyl)-D-valinate], Bifenthrin [(2-methylbiphenyl-3-ylmethyl) (Z)-(1RS)-cis-3-(2-chloro-3,3,3-trifluoroprop-1-enyl)-2,2-dimethylcyclopropanecarboxylate], 2-methyl-2-(4-bromodifluoromethoxyphenyl)propyl (3-phenoxybenzyl) ether, Tralomethrin [(S)-α-cyano-3-phenoxybenzyl (1R-cis)3 {(1RS)-(1,2,2,2-tetrabromoethyl)} -2,2-dimethylcyclopropanecarboxylate], Silafluofen [4-ethoxyphenyl {3-(4-fluoro-3-phenoxyphenyl)propyl} dimethylsilane], d-Phenothrin [3-phenoxybenzyl (1R-cis,trans)-chrysanthemate], Cyphenothrin [(RS)-α-cyano-3-phenoxybenzyl (1R-cis,trans)-chrysanthemate], d-Resmethrin [5-benzyl-3-furylmethyl (1R-cis,trans)-chrysanthemate], Acrinathrin [(S)-α-cyano-3-phenoxybenzyl (1R-cis(Z))-(2,2-dimethyl-3-{3-oxo-3-(1,1,1,3,3,3-hexafluoropropyloxy)propenyl} cyclopropanecarboxylate], Cyfluthrin [(RS)-α-cyano-4-fluoro-3-phenoxybenzyl 3-(2,2-dichlorovinyl)-2,2-dimethylcyclopropanecarboxylate], Tefluthrin [2,3,5,6-tetrafluoro-4-methylbenzyl (1RS-cis(Z))-3-(2-chloro-3,3,3-trifluoroprop-1-enyl)-2,2-dimethylcyclopropanecarboxylate], Transfluthrin [2,3,5,6-tetrafluorobenzyl (1R-trans)-3-(2,2-dichlorovinyl)-2,2-dimethylcyclopropanecarboxylate], Tetramethrin [3,4,5,6-tetrahydrophthalimidomethyl (1RS)-cis,trans-chrysanthemate], Allethrin [(RS)-3-allyl-2-methyl-4-oxocyclopent-2-enyl (1RS)-cis,trans-chrysanthe-mate], Prallethrin [(S)-2-methyl-4-oxo-3-(2-propynyl)cyclopent-2-enyl (1R)-cis,trans-chrysanthemate], Empenthrin [(RS)-1-ethynyl-2-methyl-2-pentenyl (1R)-cis,trans-chrysanthemate], Imiprothrin [2,5-dioxo-3-(prop-2-ynyl)imidazolidin-1-ylmethyl (1R)-cis,trans-2,2-dimethyl-3-(2-methylprop-1-enyl)cyclopropanecarboxylate], d-Flamethrin [5-(2-propynyl)furfuryl (1R)-cis, trans-2,2-chrysanthemate], 5-(2-propynyl)furfuryl 2,2,3,3-tetramethylcyclopropanecarboxylate]; N-cyanoamidine derivatives such as N-cyano-N'-methyl-N'-(6-chloro-3-pyridylmethyl)acetoamidine; chlorinated hydrocarbon compounds such as Endosulfan [6,7,8,9,10,10-hexachloro-1,5,5a,6,9,9a-hexahydro-6,9-methano-2,4,3-benzodioxathiepin oxide], γ-BHC [1,2,3,4,5,6-hexachlorocyclohexane], 1,1-bis(chlorophenyl)-2,2,2-trichloroethanol; benzoylphenylurea type compounds such as Chlorfluazuron [1-(3,5-dichloro-4-(3-chloro-5-trifluoromethylpyridin-2-yloxy)phenyl)-3-(2,6-difluorobenzoyl)urea], Teflubenzron [1-(3,5-dichloro-2,4-difluorophenyl)-3-(2,6-difluorobenzoyl)urea], Flufenoxuron [1-(4-(2-chloro-4-trifluoromethylphenoxy)-2-fluorophenyl)-3-(2,6-difluorobenzoyl)urea]; thiourea derivatives such as Diafenthiuron [N-(2,6-diisopropyl-4-phenoxyphenyl)-N'-tert-butylcarbodiimide]; Phenylpyrazole type compounds; Metoxadiazon [5-methoxy-3-(2-methoxyphenyl)-1,3,4-oxadiazol-2-(3H)-one], Bromopropylate [isopropyl 4,4'-dibromobenzilate], Tetradifon [4-chlorophenyl 2,4,5-trichlorophenylsulfone], Quinomethionate [S,S-6-methylquinoxaline-2,3-diyldithiocarbonate], Pyridaben [2-ter-butyl-5-(4-tert-butylbenzylthio)-4-chloropyridazin-3(2H)-one], Fenpyroximate [tert-butyl(E)-4-[(1,3-dimethyl-5-phenoxypyrazol-4-yl)methyleneaminoxymethyl] benzoate], Tebufenpyrad [N-4-tert-butylbenzyl)-4-chloro-3-ethyl-1-methyl-5-pyrazolecarboxamide], Polynactin complexes [tetranactin, dinactin and trinactin], Pyrimidifen [5-chloro-N-[2-{4-(2-ethoxyethyl)-2,3-dimethylphenoxy}ethyl]-6-ethylpyrimidine-4-amine], Milbemectin, Abamectin, ivermectin, azadirachtin [AZAD], etc.

The repellents include, for example, 3,4-caranediol, N,N-diethyl-m-toluamide, 1-methylpropyl 2-(2-hydroxyethyl)-1-piperidinecarboxylate, p-menthane-3,8-diol, and plant essential oils such as hyssop oil.

The synergists include, for example, bis-(2,3,3,3-tetrachloropropyl) ether (S-421), N-(2-ethylhexyl)bicyclo [2.2.1]hept-5-ene-2,3-dicarboximide (MGK-264), and α-[2-(2-butoxyethoxy)ethoxy]-4,5-methylenedioxy-2-propyltoluene (piperonyl butoxide).

When the present compound is used as an active ingredient of a composition for controlling pest for preventing domestic epidemics or for controlling pest for animals, formulations in the form of emulsifiable concentrates, wettable powders or flowable concentrates are applied usually after having been diluted with water so that the formulations have a concentration of the present compound falling within the range of from 0.1 to 10,000 ppm. Formulations in the form of oil formulations, aerosols, fumigants, smoking formulations, volatile formulations, foggings, ULV formulations, poisonous baits or resin formulations are applied as they are.

Both the applying dosage and the applying concentration of the above formulations can be properly determined depending on the conditions such as the type of formulation, when, where and how these formulations are applied, kind of pests, degree of damage, etc.

The present compound exhibits a marked controlling effect on pests when vaporized by heating or without heating. Therefore, it is particularly effective as an active ingredient of a composition for controlling insect pest for preventing epidemics.

Examples

The present invention is illustrated with reference to the following production example, formulation examples and test examples, which should not be construed as limiting the scope of the invention.

Firstly, production of the present compound is exemplified.

Production Example

11.4 Grams of (1R)-trans-3-(2,2-dichlorovinyl)-2,2-dimethylcyclopropanecarboxylic acid chloride was added to a mixture of 9.7 g of 4-methyl-2,3,5,6-tetrafluorobenzyl alcohol, 4.3 g of pyridine and 200 ml of tetrahydrofuran under ice-cooling. The resulting mixture was allowed to react at room temperature for 8 hours. Then, the resultant reaction solution was poured into about 400 ml of ice water and extracted twice with 400 ml of ethyl acetate. The combined ethyl acetate layer was washed with a saturated aqueous sodium chloride solution, dried over anhydrous sodium sulfate and then concentrated under reduced pressure to obtain a crude oil. The obtained crude oil was purified by a silica gel column chromatography to obtain 17.0 g (yield: 88%) of 4-methyl-2,3,5,6-tetrafluorobenzyl (1R)-trans-3-(2,2-dichlorovinyl)-2,2-dimethylcyclopropanecarboxylate (the present compound).

$^1$H-NMR ($CDCl_3$, TMS internal standard) δ values (ppm) : 1.20(s, 3H), 1.30(s, 3H), 1.60(d, 1H), 2.25(dd, 1H), 2.30(s, 3H), 5.23(s, 2H), 5.60(d, 1H).

The present compound obtained above is essentially free from (1R)-cis isomer and is utilized for Test Examples below.

Next, formulation examples are described below. In the formulation examples, parts are all by weight.

Formulation Example 1

A 20% emulsifiable concentrate is obtained by dissolving 20 parts of the present compound in 65 parts of xylene, adding thereto 15 parts of an emulsifier Sorpol 3005X (a registered trade name, Toho Chemical Co., Ltd.), and thoroughly stirring and mixing the resultant mixture.

Formulation Example 2

A 40% wettable powder is obtained by thoroughly mixing 40 parts of the present compound with 5 parts of Sorpol 3005X (described above), adding thereto 32 parts of Carplex #80 (a registered trade name, Shionogi & Co., Ltd.; fine powder of synthetic hydrated silicon dioxide) and 23 parts of 300-mesh diatomaceous earth, and stirring and

mixing the resultant mixture in a juice mixer.

Formulation Example 3

1.5% Granules are obtained by thoroughly mixing 1.5 parts of the present compound with 98.5 parts of AGSORBLVM-MS 24/48 (a calcined product of montmorillonite, mfd. by OIL DRI Corp.; a granular carrier having a particle size of 24 to 48 mesh).

Formulation Example 4

A mixture of 10 parts of the present compound, 10 parts of phenylxylylethane and 0.5 parts of Sumidur L-75 (tolylene diisocyanate, mfd. by Sumitomo Bayer Urethane Comp., Ltd.) is added to 20 parts of a 10% aqueous gum arabic solution. The resulting mixture is stirred in a homomixer to obtain an emulsion having an average particle size of 20 μm. Then, 2 parts of ethylene glycol is added to the emulsion. The resultant mixture is allowed to react on a hot bath at 60°C for 24 hours to obtain a microcapsule slurry. On the other hand, 0.2 part of xanthan gum and 1.0 part of Veegum R (aluminum magnesium silicate, mfd. by Sanyo Chemical Industries Ltd.) are dispersed in 56.3 parts of ion-exchanged water to obtain a thickening agent solution.

42.5 Parts of the microcapsule slurry prepared above and 57.5 parts of the thickening agent solution prepared above are mixed to obtain 10% microcapsules.

Formulation Example 5

A mixture of 10 parts of the present compound and 10 parts of phenylxylylethane is added to 20 parts of a 10% poly(ethylene glycol) aqueous solution. The resulting mixture is stirred in a homomixer to obtain an emulsion having an average particle size of 3 μm. On the other hand, 0.2 part of xanthan gum and 1.0 part of Veegum R (aluminum magnesium silicate, mfd. by Sanyo Chemical Industries Ltd.) are dispersed in 58.8 parts of ion-exchanged water to obtain a thickening agent solution.

40 Parts of the emulsion prepared above and 60 parts of the thickening agent solution prepared above are mixed to obtain a 10% flowable concentrate.

Formulation Example 6

A 5% dust is obtained by stirring and mixing 5 parts of the present compound, 3 parts of Carplex #80 (a registered trade name, Shionogi & Co., Ltd.; fine powder of synthetic hydrated silicon dioxide), 0.3 part of PAP and 91.7 parts of 300-mesh talc in a juice mixer.

Formulation Example 7

A 0.1% oil formulation is obtained by dissolving 0.1 part of the present compound in 5 parts of dichloromethane and mixing the resulting solution in 94.9 parts of deodorized kerosene.

Formulation Example 8

An oil-based aerosol is obtained by mixing 1 part of the present compound, 5 parts of dichloromethane and 34 parts of deodorized kerosene to obtain a solution, charging the solution into an aerosol container, attaching a valve part to the container, and then compressing 60 parts of a propellant (liquefied petroleum gas) into the container under pressure through the valve part.

Formulation Example 9

A water-based aerosol is obtained by mixing 0.6 part of the present compound, 5 parts of xylene, 3.4 parts of deodorized kerosene and 1 part of an emulsifier {Atmos 300 (a registered trade name, Atlas Chemical Corp.)} to obtain a solution, charging the solution with 50 parts of pure water into an aerosol container, attaching a valve part to the container, and then compressing 40 parts of a propellant (liquefied petroleum gas) into the container under pressure through the valve part.

Formulation Example 10

A mosquito coil is obtained by dissolving 0.3 g of the present compound in 20 ml of acetone, stirring and mixing uniformly the resulting solution with 99.7 g of a carrier for mosquito coil (a mixture of Tabu powder, Pyrethrum marc and wood powder in the ratio of 4:3:3), adding thereto 120 ml of water, thoroughly kneading the resulting mixture, and molding and drying the kneaded mixture.

Formulation Example 11

Acetone is added to a mixture of 0.8 g of the present compound and 0.4 g of piperonyl butoxide to prepare a solution having a total volume of 10 ml. A base material for electric mat (a plate obtained by coagulating fibril of a mixture of cotton linter and pulp) having an area of 2.5 cm x 1.5 cm and a thickness of 0.3 cm is uniformly impregnated with 0.5 ml of the solution prepared above, to obtain an electric mosquito mat.

Formulation Example 12

A part used for an absorbent-wick type heating vaporization device is obtained by dissolving 3 parts of the present compound in 97 parts of deodorized kerosene to obtain a solution, placing the solution in a container made of vinyl chloride, and inserting one end of an absorbent wick (obtained by coagulating inorganic powder with a binder and baking the coagulated powder) into the container so that the other end of the wick can be heated with a heater.

Formulation Example 13

A heating smoking formulation is obtained by dissolving 100 mg of the present compound in an adequate amount of acetone to obtain a solution, and impregnating a porous ceramic plate having an area of 4.0 cm square and a thickness of 1.2 mm with the solution.

Formulation Example 14

A non-heating volatile formulation is obtained by dissolving 100 μg of the present compound in an adequate amount of acetone, applying the resulting solution uniformly on a filter paper having an area of 2 cm square and a thickness of 0.3 mm, and then air-drying the filter paper to remove the acetone.

Formulation Example 15

An acarine-controlling sheet is obtained by impregnating a filter paper with a solution of the present compound in acetone so that the filter paper carries the present compound in an amount of 1 g per square meter, and air-drying the filter paper to remove the acetone.

Test Example 1

A base material for mosquito coil (obtained by stirring a 4:3:3 mixture of Tabu powder, Pyrethrum marc and wood powder, adding water thereto, thoroughly kneading the resulting mixture, and molding and drying the kneaded mixture) was uniformly impregnated with a solution of the present compound in acetone so that the material contains a predetermined amount of the compound. The resultant base material was air-dried to obtain a mosquito coil.

Ten adult houseflies (Musca domestica) (male/ female = 5/5) were released in a glass chamber (70cm cube, capacity: 0.34 $m^3$). 0.3 Gram of a mosquito coil containing the present compound prepared according to the procedure described above was set on a holder in the center of the bottom of the chamber, ignited at one end thereof, and taken out of the chamber after 30 seconds of combustion. The knocked-down houseflies were counted in ten minutes after the setting of the coil. In 25 minutes after the setting of the coil, the test insects were recovered into a cup for recovery and given water and diet.

The dead and alive were counted in 24 hours. Two replications of the test were carried out. The same test procedure was repeated for 4-methyl-2,3,5,6-tetrafluorobenzyl (1R)-cis-3-(2,2-dichlorovinyl)-2,2-dimethylcyclopropanecarboxylate (hereinafter referred to as reference compound A) and for a mixture of 50% by weight of 4-methyl-2,3,5,6-tetrafluorobenzyl (1RS)-cis-3-(2,2-dichlorovinyl)-2,2-dimethylcyclopropanecarboxylate and 50% by weight of 4-methyl-2,3,5,6-tetrafluorobenzyl (1RS)-trans-3-(2,2-dichlorovinyl)-2,2-dimethylcyclopropanecarboxylate (this mixture is hereinafter referred to as reference compound B). Table 1 shows the results. In the table, the knocking-down activity and the lethal activity are indicated according to the following criterion:

Excellent: All the insects were knocked down or dead (the total in the two replications).
Good: 1 to 2 insects were not knocked down or dead (ditto).
Mediocre: 3 to 4 insects were not knocked down or dead (ditto).
Bad: 5 or more insects were not knocked down or dead (ditto).

Table 1

| Test compound | Concentration (%W/W) | Knocking-down activity | Lethal activity |
|---|---|---|---|
| Present compound | 0.5 | Excellent | Good |
| Reference compound A | 0.5 | Bad | Bad |
| Reference compound B | 0.5 | Bad | Bad |
| Reference compound B | 1.0 | Mediocre | Bad |

Test Example 2

- Insecticidal activity against housefly

The present compound was diluted with acetone to concentrations of 100, 50, 25, 12.5 and 6.25 ppm. Each of reference compounds A and B was also diluted with acetone to concentrations of 400, 200, 100, 50 and 25 ppm. Each of the dilutions thus prepared was applied on the nota of female adult houseflies in a volume of 0.5 µl per insect. The insects were given water and diet. After 24 hours, the dead and alive were counted for mortality. From the mortality, $LD_{50}$ (mg/kg) value was determined.

- Toxicity to rat

Each of the present compound and reference compounds A and B was diluted with corn oil to predetermined concentrations. Each of the dilutions thus prepared was administered to male rats in an amount of 10 ml/kg. The rats were given water and diet. After 7 days, the dead and alive were counted for mortality. From the mortality, $LD_{50}$ (mg/kg) value was determined.

- Calculation of safety factor

The safety factor was calculated from the insecticidal activity against housefly and the toxicity to rat according to the following equation. Table 2 shows the results.

Safety factor = Toxicity to rat ($LD_{50}$ value)] /

[Insecticidal activity against housefly

($LD_{50}$ value)]

Table 2

| Test compound | Insecticidal activity against housefly ($LD_{50}$) | Toxicity to rat ($LD_{50}$) | Safety factor |
|---|---|---|---|
| Present compound | 0.93 | 300 | 320 |
| Reference compound A | 2.8 | <100 | <36 |
| Reference compound B | 2.9 | <100 | <35 |

The present compound is excellent in controlling effect on insect pests and safety to mammals and hence is very effective as an active ingredient of compositions for controlling insect pest, in particular, compositions for controlling insect pest for preventing epidemics.

## Claims

1. 4-methyl-2,3,5,6-tetrafluorobenzyl (1R)-trans-3-(2,2-dichlorovinyl)-2,2-dimethylcyclopropanecarboxylate represented by the formula:

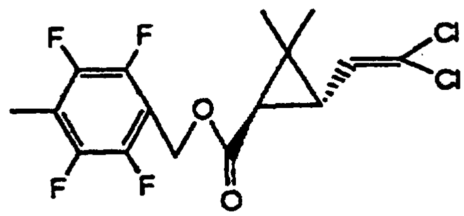

2. A composition for controlling arthropods, which comprises the compound of Claim 1 as an active ingredient and an inert carrier.

3. A method for controlling arthropods, which comprises applying an effective amount of the compound of claim 1 to the arthropods or a locus which the arthropods inhabit.

4. Use of the compound of claim 1 for controlling arthropods.

## Patentansprüche

1. (4-Methyl-2,3,5,6-tetrafluorbenzyl)-(1R)-trans-3-(2,2-dichlorvinyl)-2,2-dimethylcyclopropancarboxylat der Formel:

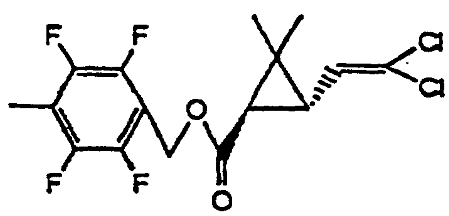

2. Zusammensetzung zur Bekämpfung von Gliederfüßlern, die die Verbindung nach Anspruch 1 als Wirkstoff und einen inerten Träger umfasst.

3. Verfahren zur Bekämpfung von Gliederfüßlern, das die Applikation einer wirksamen Menge der Verbindung nach Anspruch 1 auf die Gliederfüßler oder einen Ort, den die Gliederfüßlern bewohnen, umfasst.

4. Verwendung der Verbindung nach Anspruch 1 zur Bekämpfung von Gliederfüßlern.

## Revendications

1. (1R)-trans-3-(2,2-dichlorovinyl)-2,2-diméthyl-cyclopropanecarboxylate de 4-méthyl-2,3,5,6-tétrafluorobenzyle représenté par la formule :

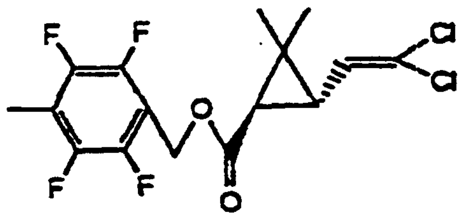

**2.** Composition pour lutter contre des arthropodes, qui comprend le composé de la revendication 1 en tant qu'ingrédient actif et un véhicule inerte.

**3.** Procédé pour lutter contre des arthropodes, qui comprend l'application d'une quantité efficace du composé de la revendication 1 aux arthropodes ou à un lieu habité par les arthropodes.

**4.** Utilisation du composé de la revendication 1 pour lutter contre les arthropodes.